(19) **Europäisches Patentamt**
**European Patent Office**
**Office européen des brevets**

(11) **EP 2 918 295 B1**

(12) **EUROPÄISCHE PATENTSCHRIFT**

(45) Veröffentlichungstag und Bekanntmachung des Hinweises auf die Patenterteilung:
**17.01.2018 Patentblatt 2018/03**

(51) Int Cl.:
*A61L 9/04* (2006.01)     *A01M 1/20* (2006.01)
*A61L 9/12* (2006.01)     *G01N 33/00* (2006.01)
*A01N 25/02* (2006.01)

(21) Anmeldenummer: **14158779.0**

(22) Anmeldetag: **11.03.2014**

(54) **DUFTFREISETZUNGSSYSTEM MIT OPTISCHER ENDPUNKTSANZEIGE**

RELEASE SYSTEM FOR FRAGRANCES WITH OPTICAL END-OF-LIFE INDICATOR

DISPOSITIF DE LIBÉRATION DE PARFUMS AVEC INDICATEUR DE FIN DE VIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**16.09.2015 Patentblatt 2015/38**

(73) Patentinhaber: **Symrise AG**
**37603 Holzminden (DE)**

(72) Erfinder:
• **Wiedemann, Jörn**
**37603 Holzminden (DE)**

• **Rube, Jennifer**
**37671 Höxter (DE)**
• **Müller, Dirk**
**37586 Dassel (DE)**

(74) Vertreter: **Fabry, Bernd**
**IP2 Patentanwalts GmbH**
**Schlossstrasse 523**
**41238 Mönchengladbach (DE)**

(56) Entgegenhaltungen:
**WO-A1-2004/087225**

EP 2 918 295 B1

**Beschreibung**

**GEBIET DER ERFINDUNG**

Die Erfindung befindet sich auf dem Gebiet Duftstofffreisetzung und betrifft ein Freisetzungssystem, bei dem die Erschöpfung des Wirkstoffes durch eine Phasentrennung angezeigt wird, ein entsprechendes Verfahrens sowie eine Verwendung.

**STAND DER TECHNIK**

Zur Beduftung und Erfrischung von Räumen werden neben Versprühsystemen insbesondere Verdunstungssysteme verwendet. Diese Verdunstungssysteme bestehen in der Regel aus Trägermaterialien, sehr einfachen Behältern sowie sehr speziellen Behältern mit Hilfsmitteln, aus denen die Duftstoffe an die Raumluft abgegeben werden. Seit langer Zeit ist man bestrebt, dem Verbraucher durch ein visuelles Signal anzuzeigen, dass der Vorrat der Duftstoffe verbraucht ist.

In der US 4,128,508 löst man diese Aufgabe durch ein Indikatorsystem enthaltend einen pH-Indikator und eine langsam verdunstende Säure oder Base. Durch die Änderung des pH-Wertes der Duftstoffmischung zeigt die veränderte Farbe des pH-Indikators das Nutzungsende an.

WO 2003 031 966 A1 beschreibt ein System enthaltend flüchtige Farben. Durch das Verdunsten der flüchtigen Farbe wird eine Farbänderung erzielt, die den Verbrauch der Duftstoffe signalisiert. Nachteilig an beiden Systemen ist, dass die Farbänderung nicht durch die Duftstoffe selbst hervorgerufen wird, sondern durch Hilfsstoffe. Es ergibt sich zur Erzielung einer verlässlichen Nutzungsendpunktanzeige die Notwendigkeit, die Duftstoffe so auszuwählen, dass diese genau so schnell verdunsten wie die Hilfsstoffe. Die Darstellung eines kreativen und komplexen Duftes wird dadurch unmöglich oder zumindest erheblich erschwert.

GB 2444702 A offenbart nicht wässrige Mischungen enthaltend Halochrome, Duftstoffe, nicht flüchtige Säuren oder Basen sowie hochpolare, schwerflüchtige Lösungsmittel. Nachteilig an einem solchen System sind einerseits die verwendeten Halochrome, welche über sehr reaktive Atome, beispielsweise Halogenatome oder reaktive Gruppen, beispielsweise Aminogruppen, verfügen. Daneben ist die Anwesenheit von Säuren oder Basen erforderlich, welche ein günstiges Milieu für Reaktionen schaffen. Infolge dieser ungünstigen Bedingungen neigen diese Systeme zu vielfältigen Reaktionen, so dass sowohl die Duftstoffe als auch die Halochrome abgebaut werden und bereits nach kurzer Zeit eine Verringerung der Geruchsintensität bzw. die Bildung von Fehlgerüchen als auch ein Verblassen der Farbe zu beobachten sind. Für eine wirtschaftliche Nutzung ist jedoch eine sehr lange Lagerstabilität der Duftstoffsysteme erforderlich, beispielsweise wegen langer Lagerzeiten beim Vertrieb der Raumdüfte.

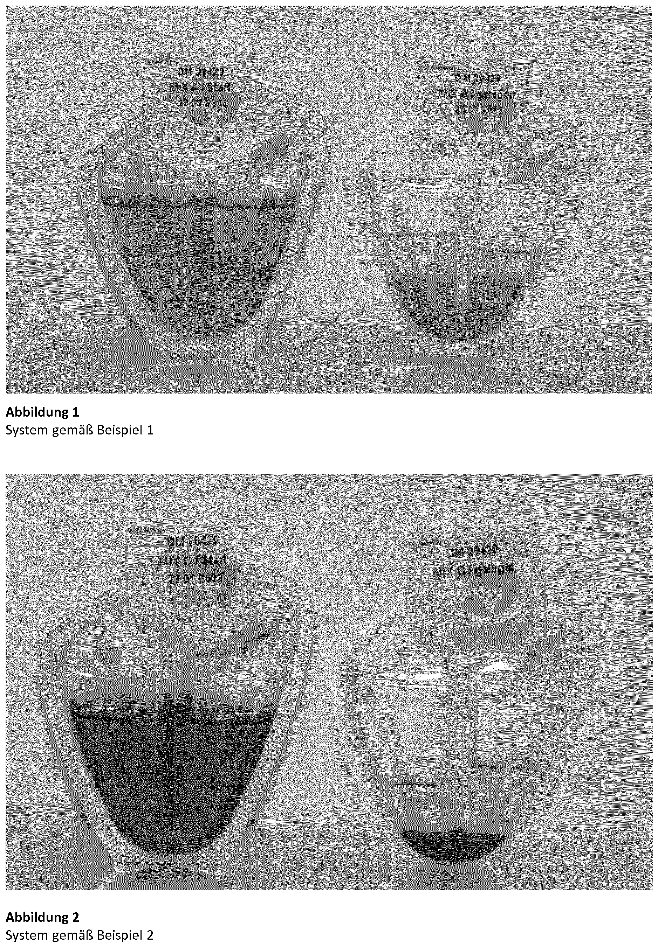

In der WO 2004/087225 A1 (siehe Abb. 1) geht es um eine Vorrichtung zur Ausbreitung eines Luftbehandlungsmittels, wie z.B. ein Lufterfrischer, ein Insektizid oder ein Geruchsneutralisator, in einer Umgebungsatmosphäre. Die Vorrichtung umfasst einen Behälter (6), in dem eine wässrige Phase (7) und eine nicht mischbare flüssigen Phase (8) gehalten wird. Diese nicht mischbare flüssige Phase (8) enthält das Luftbehandlungsmittel, welches mittels des Dochtes (10) in der Außenseite des Behälters freigesetzt wird. Die nicht mischbare Phase umfasst vorzugsweise ein flüchtiges Silikon.

Eine Alternative könnten Indikator-Systeme mit so genanntem "time point" darstellen, bei denen nach Ablauf einer bestimmten Zeit eine Farbänderung eintritt. Diese sind für Duftdispenser jedoch nicht geeignet, da die Verdampfungsgeschwindigkeit stark von der Umgebungstemperatur und anderen Faktoren beeinflusst wird. Bei unterschiedlichen Bedingungen wird der Endpunkt nicht zur gleichen Zeit eintreten.

Die Aufgabe der vorliegenden Erfindung hat daher in erster Linie darin bestanden, Duftstoffsysteme mit Nutzungsendpunktanzeige zur Verfügung zu stellen, die die eingangs geschilderten Nachteile des Stands der Technik vermeiden. Die Systeme sollten sich dadurch auszeichnen, dass die Endpunkanzeige nicht durch Verringerung der Konzentration eines Hilfsstoffs bewirkt wird. Zudem bestand eine weitere Teilaufgabe darin, besonders lagerstabile Systeme zur Verfügung zu stellen.

**BESCHREIBUNG DER ERFINDUNG**

Gegenstand der Erfindung ist ein Wirkstofffreisetzungssystem mit optischer Endpunktsanzeige, umfassend

   (a) einen Behälter zur Aufnahme der Komponenten,
   (b) zwei darin befindliche Lösungsmittel A und B, die nicht oder nur geringfügig miteinander mischbar sind,
   (c) mindestens einen Wirkstoff, der als Lösungsvermittler für die Lösungsmittel A und B dient, wobei eine einphasige Zubereitung entsteht, sowie
   (d) einen Mechanismus, der die Freisetzung des Wirkstoffes in die Umgebung ermöglicht, jedoch die anderen volatilen Bestandteile der Zusammensetzung zurück hält,

wobei die Wirkstoffe Duftstoffe, antimikrobielle Wirkstoffe und/oder Insektenrepellentien sind, und

wobei die mischbare Lösungsmittel A und B mit einer solchen Menge genannten Wirkstoffes versetzt wird, dass eine einphasige Zubereitung entsteht,

und wobei durch die Erschöpfung des genannten Wirkstoffes die Phasen sich entmischen.

Überraschenderweise wurde gefunden, dass sich spezielle Aktivstoffe, speziell Duftstoffe, als Phasenvermittler für ansonsten nicht oder kaum miteinander mischbare Lösungsmittel eignen. Auf diese Weise lassen sich einphasige Systeme mit hoher Lagerstabilität erhalten. Sinkt die Konzentration des Wirkstoffs unter eine kritische Konzentration, beispielsweise durch Verdampfung, kann er nicht länger als Phasenvermittler wirken und es kommt zur Entmischung. Diese Entmischung dient dann als optische Anzeige, dass das System seine Menge an ursprünglich enthaltenem Wirkstoff an die Umgebung abgegeben hat - was als "optische Endpunktsanzeige" bezeichnet wird.

Der große Vorteil dieses Systems gegenüber dem Stand der Technik besteht darin, dass die Konzentration des Wirkstoffs selbst eine direkte Endpunktsanzeige liefert, es ist also nicht erforderlich, einen Hilfsstoff einzusetzen, dessen Abnahme gemessen wird und dabei mehr oder weniger genau mit der jeweiligen Konzentration des eigentlichen Wirkstoffs korrelieren muss (indirekte Endpunktsanzeige).

Für die vorliegende Erfindung ist zudem auch die Anwesenheit von Säuren oder Basen nicht erforderlich, diese Tatsache ist besonders für die Stabilität der Zusammensetzung von enormer Bedeutung.

## LÖSUNGSMITTEL

Bei den Lösungsmitteln handelt es sich um beliebige Stoffe in flüssiger Form, die in der Lage sind mit dem Aktivstoff eine homogene Phase zu bilden. Wird das volatile Lösungsmittel in dem Behälter festgehalten, so kann es sich auch um einen bekannten Duftstoff handeln. Durch das Verhindern des Austritts des Stoffes mit eigener geruchlichen Performance begrenzt sich die Funktion des Duftstoffs nur auf das Lösen der anderen Bestandteile und wird in der vorliegenden Erfindung als Lösungsmittel eingesetzt.

Der Anteil der Lösungsmittel A und B ist vorzugsweise so einzustellen, dass nach der Erschöpfung des Aktivstoffs eine ausreichende Menge der Lösungsmittel zurückbleibt, so dass die Phasentrennung auch deutlich zu erkennen ist.

Gleichfalls sind die beiden Lösungs-Komponenten auch so auszuwählen, dass sie sich in Gegenwart des Aktivstoffs erst dann entmischen, wenn dieser fast vollständig aufgebraucht ist.

Eine Vorhersage zu dem Mischungsverhalten der Lösungsmittel kann mit Hilfe von M-Zahlen ("*miscibility number*"), einem von Godfrey N.B.[1] entwickeltem Zahlensystem erfolgen.

Dieses besteht aus 31 Klassen unterteilt zwischen geringer und hoher Lipophilizität. Die Klassen werden »Miscibility Numbers« genannt. Jede Klasse besteht aus Lösungsmitteln, die ein ähnliches Mischungsverhalten mit Lösungsmitteln einer anderen Klasse zeigen. Zu dem Lösungsmittel ist eine M-Zahl angegeben und ein M-Zahlen-Bereich in welchem sich dieses Lösungsmittel löst.

[1] Godfrey N.B.:"Solvent selection via miscibility number." Chemtech. 6(1972) 359 - 363

Dipropylenglycol hat eine M-Zahl = 11 und ist mit Lösungsmitteln im Bereich zwischen M = 1 bis 26 mischbar. Ein geeignetes Partner-Lösungsmittel für das System sollte eine M-Zahl > 26 haben.

Propylencarbonat hat zwei M-Zahlen, nämlich 9 für den polaren und 17 für den unpolaren Bereich und ist daher mit Lösungsmitteln im Bereich zwischen M = 2 bis 24 mischbar. Ein geeignetes Partner-Lösungsmittel für das System sollte eine M-Zahl > 24 haben.

Liegt keine genaue Angabe zu dem Mischungsbereich vor, kann eine allgemeine Regel angewendet werden, die besagt: "Beträgt die Differenz der M-Zahlen der beiden Stoffe ≥16, sind die Stoffe nicht miteinander mischbar."

Die Differenz der M-Zahlen der ausgewählten Lösungsmittel soll daher mind. 16, vorzugsweise jedoch höher als 16 sein.

Ist die Differenz der M-Zahlen genau 16, besteht die Möglichkeit, dass sich die Lösungsmittel bei einer Umgebungstemperatur > 25°C lösen und höchst wahrscheinlich bei einer Umgebungstemperatur > 75°C lösen. Solche Systeme sind möglicherweise für eine Anwendung bei Raumtemperatur geeignet, sollen jedoch für Anwendungen bei höheren Temperarturen vermieden werden, wie z.B. bei einem Spülmaschinen Air Freshener oder bei einem elektrischen Air Freshener, wo die Zusammensetzung zur besseren Verdampfung erwärmt wird)

Die flüchtige Duftkomponente kann auf den M - Zahlen-Bereich zwischen 13 und 26 beschränkt werden. Beide ausgewählten Lösungsmittel sollen in diesem Bereich löslich sein d.h. die Differenz der M-Zahlen zwischen Lösungsmittel und der Flüchtigen Duftkomponente soll max. 15, vorzugsweise jedoch unter 15 sein.

Liegt keine Angabe zu der M-Zahl einer Flüssigkeit, kann diese bestimmt werden. Zur Bestimmung der M-Zahl wird zu untersuchende Flüssigkeit nach einander zu den Standartlösungsmitteln gegeben und nach 30min bei RT die Löslichkeit beurteilt. Zu jeder M-Zahl-Klasse gibt es ein Standardlösungsmittel, insgesamt 31 M-Zahl-Klassen und Standardlösungsmittel. Zur Berechnung der M-Zahl wird die oben genannte allgemeine Regel angewendet, die besagt: "Beträgt die Differenz der M-Zahlen der beiden Stoffe ≤15, sind die Stoffe miteinander mischbar." In der folgenden Tabelle A sind exemplarisch Bestimmungen der M-Zahl von Phenoxyethanol, Propylacetat und Isobutylisobutyrat angegeben:

**Tabelle A**

| M-Zahlen | | | | |
|---|---|---|---|---|
| | M-Zahl bekannt [1] | 12 | 19 | 23 |
| | M-Zahl berechnet (+15) | 27 - 15 = 12 | 4 + 15 = 19 | 8+ 15=23 |
| M-Zahl | Lösungsmittel | Phenoxethanol | Propylacetat | Isobutylisob. |
| | | | | |
| 1 | Glyerin | klare Lösung | 2-phasig | 2-phasig |
| 2 | Ethylenglycol | klare Lösung | 2-phasig | 2-phasig |
| 3 | 1,4-Butanediol | klare Lösung | 2-phasig | 2-phasig |
| 4 | 1.2-PROPYLENE GLYCOL | klare Lösung | klare Lösung | 2-phasig |
| 5 | DIETHYLENE GLYCOL | klare Lösung | klare Lösung | 2-phasig |
| 6 | TRIETHYLENE GLYCOL | klare Lösung | klare Lösung | 2-phasig |
| 7 | Tetraethylene glycol | klare Lösung | klare Lösung | 2-phasig |
| 8 | Methoxyessiqsäure | klare Lösung | klare Lösung | klare Lösung |
| 9 | Dimethylsulfoxide | klare Lösung | klare Lösung | klare Lösung |
| 10 | 2-Pyrrolidone | klare Lösung | klare Lösung | klare Lösung |
| 11 | DPG | klare Lösung | klare Lösung | klare Lösung |
| 12 | Phenoxyethanol | klare Lösung | klare Lösung | klare Lösung |
| 13 | Benzylalkohol | klare Lösung | klare Lösung | klare Lösung |
| 14 | Ethanol | klare Lösung | klare Lösung | klare Lösung |
| 15 | Isopropanol | klare Lösung | klare Lösung | klare Lösung |
| 16 | Butylglycol | klare Lösung | klare Lösung | klare Lösung |
| 17 | Cyclohexanon | klare Lösung | klare Lösung | klare Lösung |
| 18 | 3-Pentanon | klare Lösung | klare Lösung | klare Lösung |
| 19 | Ethylacetat | klare Lösung | klare Lösung | klare Lösung |
| 20 | Dichlormethan | klare Lösung | klare Lösung | klare Lösung |
| 21 | Ethylpropionat | klare Lösung | klare Lösung | klare Lösung |
| 22 | Butylacetat | klare Lösung | klare Lösung | klare Lösung |
| 23 | Pentylacetat | klare Lösung | klare Lösung | klare Lösung |
| 24 | para Xylol | klare Lösung | klare Lösung | klare Lösung |
| 25 | Rizinusöl | klare Lösung | klare Lösung | klare Lösung |
| 26 | Diisopropylether | klare Lösung | klare Lösung | klare Lösung |
| 27 | Cycloocten- cis | klare Lösung | klare Lösung | klare Lösung |
| 28 | Cyclohexan | 2-phasig | klare Lösung | klare Lösung |
| 29 | Hexan | 2-phasig | klare Lösung | klare Lösung |
| 30 | Tetradecan | 2-phasig | klare Lösung | klare Lösung |
| 31 | Heptadecan | 2-phasig | klare Lösung | klare Lösung |

Die bevorzugten Lösungsmittel umfassen Pflanzenöle, $C_6$-$C_{22}$-Fettsäure-$C_1$-$C_6$-alkylester, $C_1$-$C_6$-Alkylcarbonate, $C_2$-$C_4$- Alkylenglykole sowie Siloxane, insbesondere Ester der Laurinsäure, Myristinsäure, Stearinsäure mit Ethanol, Propanol oder Isopropylalkohol, Ethylencarbonat, Propylencarbonat, Butylencarbonat, Ethylenglykol, Propylenglykol, Diethylenglykol, Dipropylenglykol und Decamethylcyclopentasiloxan, wobei das Mischungsverhältnis im Bereich zwischen etwa 40:60 und 60:40 Gewichtsteile und vorzugsweise etwa 50:50 Gewichtsteile liegen kann.

Ein wesentlicher Vorteil der Erfindung ist die Eignung von Pflanzenölen als Lösungsmittel für das beschriebene System. Die Pflanzenöle sind besonders umweltfreundlich und gesundheitlich unbedenklich. Die Auswahl der für Mensch und Umwelt unbedenklichen Stoffe, die in diesem Indikatorsystem eingesetzt werden, begrenzt sich nicht nur auf die Pflanzenöle. Es bestehen viele Möglichkeiten das Indikatorsystem nur aus Stoffen zusammenzustellen, die im Sinne der Richtlinie 67/548/EWG nicht zu gefährlichen Produkten zählen und keine besondere Gefahrenhinweise für Mensch und Umwelt erfordern.

Besonders bevorzugte Lösungsmittelsysteme mit Pflanzenölen sind Dipropylenglycol/ Rapsöl, Triacetin/ Sonnenblumenöl; Dipropylenglycol/Sonnenblumenöl; Triacetin/Rapsöl;

Die oben genannten Lösungsmittelsysteme können zusammen mit Lebensmittelfarbstoffen und einem natürlichen Duftöl als Aktivstoff ein besonders umweltfreundliches Air Freshener bilden.

Weitere bevorzugte Lösungsmittelsysteme sind etwa Decamethylcyclopentasiloxan/Dipropylenglykol; Isopropylmyristat/Propylencarbonat;

Sowie Glycerin/Symfresh NX; Propylenglycol 1,2/ Benzylbenzoat; Propylencarbonat/ Dioctyladipat; Triethylcitrat/ Decamethylcyclopentasiloxan (DOW CORNING 245 FLUID); Polyethylenglykol 400 (Polydiol 400) /Medizinisches Weißöl (Pionier CP65)

Zusammenstellung von weiteren geeigneten Lösungsmittelpaaren, dabei kann das Lösungsmittel A mit dem beliebigen Lösungsmittel B aus demselben Tabellenabschnitt ein Paar bilden. Die Tabellenabschnitte sind nach M-Zahl des Lösungsmittels A unterteilt.

Beispiel zu dem 2-ten Tabellenabschnitt mit Läsungsmittel A = 2:

Etylenglycol kann als Lösungsmittel A mit M=2 mit jedem Lösungsmittel B im M-Zahlenbereich von 19 bis 21 ein geeignetes Paar bilden. Das gleiche gilt für 1,2,6 - Hexantriol und 2- Hydroxyethylcarbonat. Der Tabellenabschnitt zu LM A = 2 enthält demnach 3 x 14 = 42 geeignete Lösungsmittelpaare. Insgesamt sind in der **Tabelle B** eine Vielzahl von geeigneten Lösungsmittelpaare genannt.

**Tabelle B**

| Lösungsmittelpaare | | | |
|---|---|---|---|
| M-Zahl LM A = 1 | Lösungsmittel A | Lösungsmittel B | M-Zahl LM 18 $\leq$ B $\leq$ 20 |
| | Glycerin | SymFresh NX | |
| | | Diethyl carbonate | |
| | | Dimethyladipat | |
| | | Propylenglycoldiacetat | |
| | | Benzylalcohol | |
| | | Triacetin | |
| | | Triethylcitrat | |
| | | Dimethylphthalat | |
| | | Diethylphtalat | |
| M-Zahl LM A = 2 | Lösungsmittel A | Lösungsmittel B | M-Zahl LM 19 $\leq$ B $\leq$ 21 |
| | Ethylene Glycol | Dimethyladipat | |
| | 1,2,6-Hexantriol | Diethyl carbonate | |
| | 2-Hydroxyethylcarbamat | Diallyladipat | |
| | | Diethyladipat | |

(fortgesetzt)

| M-Zahl LM A = 2 | Lösungsmittel A | Lösungsmittel B | M-Zahl LM 19 ≤ B ≤ 21 |
|---|---|---|---|
| | | Ethylbenzoat | |
| | | Methyl-iso-amylketon | |
| | | Propylenglycoldiacetat | |
| | | Methyl-iso-butylketon | |
| | | Benzylalcohol | |
| | | Benzylbenzoat | |
| | | Diethylphtalat | |
| | | Triacetin | |
| | | Triethylcitrat | |
| | | Dimethylphthalat | |
| M-Zahl LM A = 3 | Lösungsmittel A | Lösungsmittel B | M-Zahl LM 20 ≤ B ≤ 22 |
| | 1,3-Propandiol | Dibutylphthalat | |
| | 1,4-Butandiol | Dimethylsebacat | |
| | 1,5-Pentandiol | Diallyladipat | |
| | 2-Butene-1,4-diol | Ethylbenzoat | |
| | 2-Hydroxypropylcarbamat | Benzylalcohol | |
| | Glycerincarbonat | Dibutylmaleat | |
| | | 2-Octanon | |
| | | 3-Heptanon | |
| | | Allylether | |
| | | Benzylbenzoat | |
| | | Diethylphtalat | |
| M-Zahl LM A = 4 | Lösungsmittel A | Lösungsmittel B | M-Zahl LM 21 ≤ B ≤ 23 |
| | 1,2-Propandiol | Dimethylsebacat | |
| | 1,3-Butandiol | Dipropyl carbonate | |
| | | Ethylbenzoat | |
| | | 2-Octanon | |
| | | 3-Heptanon | |
| | | Dibutylmaleat | |
| | | Benzylbenzoat | |
| | | Diallyladipat | |
| M-Zahl LM A = 5 | Lösungsmittel A | Lösungsmittel B | M-Zahl LM 22 ≤ B ≤ 24 |
| | 2,5-Hexandiol | Dioctylphthalat | |
| | 3-Methoxy-l,2-propandiol | Dimethylsebacat | |
| | Diethelenglycol | Dibutylmaleat | |

(fortgesetzt)

| M-Zahl LM A = 5 | Lösungsmittel A | Lösungsmittel B | M-Zahl LM 22 ≤ B ≤ 24 |
|---|---|---|---|
| | | Dipropyl carbonate | |
| | | 2-Octanon | |
| | | 3-Heptanon | |
| M-Zahl LM A = 6 | Lösungsmittel A | Lösungsmittel B | M-Zahl LM 23 ≤ B ≤ 25 |
| | 1,2-Butanediol | Dioctylphthalat | |
| | Triethylenglycol | Rizinusöl | |
| | Ethylencarbonat | Dipropyl carbonate | |
| | Hydroxypropylmaleate | Di-iso-butylketon | |
| | Hydroxypropylmaleate | Di-iso-propylketon | |
| | | Di-iso-propylbenzol | |
| | | 1,2-Dibutoxyethan | |
| | | 1,3-Pentadien | |
| | | sec-Amylbenzol | |
| | | ABALYN D-E | |
| M-Zahl LM A = 7 | Lösungsmittel A | Lösungsmittel B | M-Zahl LM 24 ≤ B ≤ 16 |
| | ETHYLENE CARBONATE | Dioctylphthalat | |
| | Pentaethylenglycol | Rizinusöl | |
| | Tetraethylenglycol | Methyloleat | |
| | | Didecylphthalat | |
| | | Dioctyladipat | |
| | | HERCOLYN D-E | |
| | | Diethoxydimethylallan | |
| | | Dimethoxydimethylsilan | |
| | | Di-iso-propylbenzol | |
| | | 1,2-Dibutoxyethan | |
| | | 1,3-Pentadien | |
| | | sec-Amylbenzol | |
| | | ABALYN D-E | |
| | | 4- Vinylcyclohexen | |
| | | Cyclohexen | |
| | | Dicyclopentadien | |
| | | Methylstearat | |
| | | Pentylether | |
| | | 2-Octyl-Dodecanol | |

(fortgesetzt)

| M-Zahl LM A = 8 | Lösungsmittel A | Lösungsmittel B | M-Zahl LM 25 ≤ B ≤ 27 |
|---|---|---|---|
| | 1-(2-Hydroxyethoxy)-2-propanol | Rizinusöl | |
| | Acetol (hydroxy acetone) | Methyloleat | |
| | Methylcyanoacetat | Methylstearat | |
| | | Didecylphthalat | |
| | | Dioctyladipat | |
| | | Di-iso-propylbenzol | |
| | | 1,7 -Octadien | |
| | | sec-Amylbenzol | |
| | | 1,3-Pentadien | |
| | | 4-Vinylcyclohexen | |
| | | Cyclohexen | |
| | | Dicyclopentadien | |
| | | Diethoxydimethylallan | |
| | | Dimethoxydimethylsilan | |
| | | 1,2-Dibutoxyethan | |
| | | cis-4-Methyl-2-penten | |
| | | Cycloocten | |
| | | 2,2,4- Trimethyl-2-penten | |
| | | 2,4,4- Trirnethyl-1-penten | |
| | | ABALYN D-E | |
| | | HERCOLYN D-E | |
| | | 2-Octyl-Dodecanol | |
| | | Isopropylmyristat | |
| | | Isopropylpalmitat | |
| | | Ethylmyristat | |
| | | Pflanzenoel Triglycerid | |
| M-Zahl LM A = 9 | Lösungsmittel A | Lösungsmittel B | M-Zahl LM 26 ≤ B ≤ 28 |
| | PROPYLENE CARBONATE | Dioctyl Adipate | |
| | Dimethylsulfoxide | Methyloleat | |
| | Ethylenglycol-bis-methoxyacetate | Methylstearat | |
| | | Butyloleat | |
| | | Isopropylmyristat | |
| | | Isopropylpalmitat | |
| | | Ethylmyristat | |
| | | Pflanzenöl | |
| | | bevorzugtes Pflanzenöl ist Rapsöl | |

(fortgesetzt)

| M-Zahl LM A = 9 | Lösungsmittel A | Lösungsmittel B | M-Zahl LM 26 ≤ B ≤ 28 |
|---|---|---|---|
| | | bevorzugtes Pflanzenöl ist Sonnenblumenöl | |
| | | bevorzugtes Pflanzenöl ist Olivenöl | |
| | | bevorzugtes Pflanzenöl ist Mandelöl | |
| | | bevorzugtes Pflanzenöl ist Sojaöl | |
| | | bevorzugtes Pflanzenöl ist Leinsamenöl | |
| | | Dicyclopentadien | |
| | | 4- Vinylcyclohexen | |
| | | 2,2,4- Trimethyl-2-penten | |
| | | 2,4,4- Trirnethyl-1-penten | |
| | | Pentylether | |
| | | 1,7 -Octadien | |
| | | Cycloocten | |
| | | 1-Octen | |
| | | 1-Hepten | |
| | | Didecylphthalat | |
| | | Diethoxydimethylallan | |
| | | Dimethoxydimethylsilan | |
| | | HERCOLYN D-E | |
| | | 2-Octyl-Dodecanol | |
| M-Zahl LM A = 10 | Lösungsmittel A | Lösungsmittel B | M-Zahl LM 27 ≤ B ≤ 29 |
| | 1,2 Butylene carbonate | Butyloleat | |
| | Acetolacetat | 1-Hexadecen | |
| | | Isopropylmyristat | |
| | | ISOPROPYLPALMITAT | |
| | | ETHYLMYRISTAT | |
| | | Pflanzenöl | |
| | | bevorzugtes Pflanzenöl ist Rapsöl | |
| | | bevorzugtes Pflanzenöl ist Sonnenblumenöl | |
| | | bevorzugtes Pflanzenöl ist Olivenöl | |
| | | bevorzugtes Pflanzenöl ist Mandelöl | |
| | | bevorzugtes Pflanzenöl ist Sojaöl | |
| | | bevorzugtes Pflanzenöl ist Leinsamenöl | |
| | | Kokosnussöl | |
| | | 1,7 -Octadien | |
| | | 1- Tetradecen | |
| | | 1-Decen | |

(fortgesetzt)

| M-Zahl LM A = 10 | Lösungsmittel A | Lösungsmittel B | M-Zahl LM 27 ≤ B ≤ 29 |
|---|---|---|---|
| | | 1-Dodecen | |
| | | Bicyclohexyl | |
| | | Cyclooctan | |
| | | Cycloocten | |
| | | 1-Octen | |
| | | 1-Hepten | |
| | | 1-Octen | |
| | | Decan | |
| | | Dodecan | |
| | | 2,2,4- Trimethyl-2-penten | |
| | | 2,4,4- Trirnethyl-1-penten | |
| | | Octan | |
| | | Decamethylcyclopentasiloxan (DOW CORNING 245 FLUID) | |
| | | Tetrapropylen | |
| | | Triisobutylen | |
| | | Hexamethyldisoloxan | |
| M-Zahl LM A = 11 | Lösungsmittel A | Lösungsmittel B | M-Zahl LM 28 ≤ B ≤ 30 |
| | Triacetin | Butyloleat | |
| | Dipropylenglycol | Pflanzenöl | |
| | Dimethylmalonat | bevorzugtes Pflanzenöl ist Rapsöl | |
| | | bevorzugtes Pflanzenöl ist Sonnenblumenöl | |
| | | bevorzugtes Pflanzenöl ist Olivenöl | |
| | | bevorzugtes Pflanzenöl ist Mandelöl | |
| | | bevorzugtes Pflanzenöl ist Sojaöl | |
| | | 1-Hexadecen | |
| | | 1- Tetradecen | |
| | | Decamethylcyclopentasiloxan (z.B. DOW CORNING 245 FLUID) | |
| | | Kokosnussöl | |
| | | Hexamethyldisoloxan | |
| | | 1-Decen | |
| | | 1-Dodecen | |
| | | Bicyclohexyl | |
| | | Cyclooctan | |
| | | Decan | |
| | | Dodecan | |

(fortgesetzt)

| M-Zahl LM A = 11 | Lösungsmittel A | Lösungsmittel B | M-Zahl LM 28 ≤ B ≤ 30 |
|---|---|---|---|
| | | 1-Octen | |
| | | 1-Hepten | |
| | | Octan | |
| | | Decamethylcyclopentasiloxan (z.B. DOW CORNING 245 FLUID) | |
| | | Tetrapropylen | |
| | | Triisobutylen | |
| | | 1-Octadecen | |
| | | Hexadecan | |
| | | Tetradecan | |
| | | Hexamethyldisoloxan | |
| | | Heptadecan | |
| | | Ecolane 130 AN | |
| | | Isopar V | |
| M-Zahl LM A = 12 | Lösungsmittel A | Lösungsmittel B | M-Zahl LM 29 ≤ B ≤ 31 |
| | Dimethylphthalat | Decamethylcyclopentasiloxan (z.B. DOW CORNING 245 FLUID) | |
| | Tripropylenglycol | 1-Decen | |
| | Triethylcitrat | 1-Dodecen | |
| | 2-Phenoxyethanol | 1-Hexadecen | |
| | Diethylene glycol monomethyl ether | Coconut oil | |
| | Diethylenglycoldiacetat | Cyclooctan | |
| | Diethylsulfat | Bicyclohexyl | |
| | Dimethylmaleat | Hexamethyldisoloxan | |
| | Ethylene glycol monopropyl ether | Decan | |
| | Ethylenglycoldiacetat | Dodecan | |
| | Phenylacetonitril | Octan | |
| | 2,3-Butandion | 1- Tetradecen | |
| | 2,4-Pentandion | Tetrapropylen | |
| | 2,5-Hexandion | Triisobutylen | |
| | | 1-Octadecen | |
| | | Hexadecan | |
| | | Tetradecan | |
| | | Heptadecan | |
| | | Ecolane 130 AN | |
| | | Isopar V | |

(fortgesetzt)

| M-Zahl LM A = 12 | Lösungsmittel A | Lösungsmittel B | M-Zahl LM 29 ≤ B ≤ 31 |
|---|---|---|---|
| | | medizinisches Weißöl (z.B. Pionier CP 65) | |
| | | Mineralöl | |
| M-Zahl LM A = 13 | Lösungsmittel A | Lösungsmittel B | M-Zahl LM 30 ≤ B ≤ 31 |
| | 1-Phenoxy-2-propanol | 1-Octadecen | |
| | Diethylene glycol monoethyl ether | Hexadecan | |
| | Diethylphthalat (C8 Dicarbonsäureester) | Tetradecan | |
| | Ethylacetoacetat | medizinisches Weißöl (z.B. Pionier CP 65) | |
| | Ethylene glycol monomethyl ether | Mineralöl | |
| | Glycidylphenylether | Heptadecan | |
| | Methylmethoxyacetat | Ecolane 130 AN | |
| | Triethylenglycolmonomethylether | Isopar V | |
| | 2-Allyloxyethanol | | |
| | 2-Methoxyethanol | | |
| M-Zahl LM A = 14 | Lösungsmittel A | Lösungsmittel B | M-Zahl LM B =31 |
| | Hexandiol | medizinisches Weißöl (z.B. Pionier CP 65) | |
| | POLYDIOL 400 (Polyethylenglycol 400) | Paraffinoil weiß | |
| | Propylenglycoldiacetat | Mineralöl | |

Das Mischungsverhältnis kann ebenfalls wieder im Bereich zwischen etwa 40:60 und 60:40 Gewichtsteile und vorzugsweise etwa 50:50 Gewichtsteile liegen.

Der pH-Wert der erfindungsgemäßen Systeme ist vorzugsweise im neutralen Bereich einzustellen. Bevorzugt beträgt der pH-Wert der erfindungsgemäßen Mischung etwa 5 bis etwa 9 und besonders bevorzugt 6 bis 8. Die Lösungsmittelmischung kann optional Stabilisierungsmittel enthalten, Unter Stabilisierungsmitteln werden erfindungsgemäß bevorzugt Lichtschutzmittel, Antioxidantien und Emulgatoren verstanden.

## WIRKSTOFFE

Bei den Wirkstoffen der vorliegenden Erfindung handelt es sich vorzugsweise um Duftstoffe. Demzufolge stellen die erfindungsgemäßen Systeme auch vorzugsweise Dispenser dar, die geruchsabdeckende, geruchsneutralisierende oder insektenabschreckende Wirkstoffe, einschließlich antimikrobieller Wirkstoffe freisetzen.

## Duftstoffe

Bevorzugt eingesetzte Duftstoffe bzw. Parfümöle, die in die Mittel inkorporiert werden können, sind keinerlei Beschränkungen unterworfen. So können als Duftstoffe einzelne Riechstoffverbindungen, sowohl synthetische oder natürliche Verbindungen vom Typ der Ester, Ether, Aldehyde, Ketone, Alkohole ,Kohlenwasserstoffe, Säuren, Kohlensäureester, aromatische Kohlenwasserstoffe, aliphatische Kohlenwasserstoffe, gesättigte und/oder ungesättigte Kohlenwasserstoffe und Mischungen daraus verwendet werden. Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann allgemein bekannt. Die Duftketone können alle Ketone umfassen, die einen erwünschtem Duft oder ein Frischeempfinden verleihen können. Es können auch Gemische unterschiedlicher Ketone eingesetzt werden. Beispielsweise kann das Keton ausgewählt sein aus der Gruppe, bestehend aus Buccoxim, Iso jasmon, Methyl beta naphthyl keton, Moschus indanon, Tonalid/Moschus plus, Alpha-Damascon, Beta-Damascon, Delta-Damascon,Iso-Damascon, Damascenon, Damarose, Methyl-dihydrojasmonat, Menthon, Carvon, Campher, Fenchon, Alphalonen, Beta-Ionon, Dihydro-Beta-Ionon, Gamma-Methyl so genanntes Ionon, Fleuramon, Dihydrojasmon, Cis-Jasmon, Iso-E-Super, Methyl-cedrenyl-keton oder Methyl-cedrylon,Acetophenon, Methylacetophenon, Para-Methoxy-acetophenon, Methyl-beta-naphtyl-keton,Benzyl-aceton, Benzophenon, Para-Hydroxy-phenyl-butanon, Celery Keton oder Livescon,6-Isopropyldecahydro-2-naphton, Dimethyl-octenon, Freskomenth, 4-(I-Ethoxyvinyl)-3,3,5,5,-tetramethyl-cyclohexanon,Methyl-heptenon, 2-(2-(4-Methyl-3-cyclohexen-1-yl)propyl)-cyclopentanon, 1-(p-Menthen-6(2)-yl)-1-propanon,4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon,4-Damascol, Dulcinyl or Cassion, Gelson, Hexalon, Isocyclemon E, Methyl-cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran, Hedionund Gemischen davon. Bevorzugt können die Ketone ausgewählt sein aus AlphaDamascon, Delta Damascon, Iso Damascon, Carvon, Gamma-Methyl-ionon, Iso-E-Super,2,4,4,7-Tetramethyl-oct-6-en-3-on, Benzylaceton, Beta Damascon, Damascenon, Methyldihydrojasmonat, Methyl-cedrylon, Hedion und Gemischen davon.

Geeignete Duftaldehyde können beliebige Aldehyde sein, die entsprechend der Duftketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Anisaldehyd, Cymal,Ethylvanillin, Florhydral, Floralozon, Helional, Heliotropin, Hydroxycitronellal, Koavon, Laurinaldehyd, Canthoxal, Lyral, Lilial, Adoxal, Anisaldehyd, Cumal Methyl-nonyl-acetaldehyd, Citronellal, Citronellyloxyacetaldehyd, Cyclamenaldehyd, Bourgeonal, p,t-Bucinal, Phenylacetaldehyd, Undecylenaldehyd, Vanillin; 2,6,10-Trimethyl-9-undecenal, 3-Dodecen-I-al,alpha-n-Amylzimtaldehyd, 4-Methoxybenzaldehyd, Benzaldehyd, 3-(4-tert-Butylphenyl)-propanal,2-Methyl-3-(para-methoxyphenylpropanal), 2-Methyl-4-(2,6,6-trimethyl-2(1)-cyclohexen-1-yl)butanal,3-Phenyl-2-propenal, cis-/trans-3,7-Dimethyl-2,6-octadien-1-al, 3,7-Dimethyl-6-octen-I-al,[(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd,2,4-Dimethyl-3-cyclohexen-1-carboxyaldehyd, 2-Methyl-3-(isopropylphenyl)propanal, Decyl aldehyd, 2,6-Dimethyl-5-heptenal; 4-(tricyclo[5.2. 1.0 (2,6)]-decylidene-8)-butanal;Octahydro-4,7-methano-1H-indenecarboxaldehyd; 3-Ethoxy-4-hydroxybenzaldehyd, para-Ethyl-alpha,alpha-dimethylhydrozimtaldehyd, alpha-Methyl-3,4-(methylenedioxy)-hydrozimtaldehyd,3,4-Methylenedioxybenzaldehyd, alpha-n-Hexylzimtaldehyd, m-Cymene-7-carboxaldehyd,alpha-Methylphenylacetaldehyd, 7-Hydroxy-3,7-dimethyl octanal, Undecenal, 2,4,6-Trimethyl-3-cyclohexene-I-carboxaldehyd,4-(3)(4-Methyl-3-pentenyl)-3-cyclohexen-carboxaldehyd, 1-Dodecanal, 2,4-Dimethylcyclohexene-3-carboxaldehyd,4-(4-Hydroxy-4-methyl pentyl)-3-cylohexene-I-carboxaldehyd, 7-Methoxy-3,7-dimethyloctan-1-al,2-Methyl undecanal, 2-Methyl decanal, 1-nonanal, 1-Octanal, 2,6,10-Trimethyl-5,9-undecadienal,2-Methyl-3-(4-tertbutyl)propanal, 3-(4-Ethylphenyl)-2,2-Dimethylpropanal, 3-(4-Methoxyphenyl)-2-methylpropanal,Methylnonylacetaldehyd, 2-Phenylpropan-1-al, 3-Phenylprop-2-en-1-al, 3-Phenyl-2-pentylprop-2-en-1-al,3-Phenyl-2-hexylprop-2-enal, 3-(4-Isopropylphenyl)-2-methylpropan-1-al, 3-(4-Ethylphenyl)-2,2-dimethylpropan-1-a1,3-(4-tert-Butylphenyl)-2-methyl-propanal, 3-(3,4-Methylendioxyphenyl)-2-methylpropan-1-al,3-(4-Ethylphenyl)-2,2-dimethylpropanal, 3-(3-Isopropylphenyl)-butan-1-al, 2,6-Dimethylhept-5-en-1-al,Dihydrozimtaldehyd, 1-methyl-4-(4-methyl-3-pentenyl)-3-cyclohexene-1-carboxaldehyd,5 oder 6 Methoxyhexahydro-4,7-methanoindan-1 oder 2-carboxyaldehyd, 3,7-Dimethyloctan-1-al,1-Undecanal, 10-Undecen-1-al, 4-Hydroxy-3-methoxybenzaldehyd, 1-Methyl-3-(4-methylpentyl)-3-cyclohexene-carboxyaldehyd,7-Hydroxy-3,7-dimethyl-octanal; trans-4-decenal, 2,6-Nonadienal, para-Tolylacetaldehyd;4-Methylphenylacetaldehyd, 2-Methyl-4-(2,6,6-trimethyl-1-cyclohexen-1-yl)-2-butenal,ortho-Methoxyzimtaldehyd, 3,5,6-Trimethyl-3-cyclohexenecarboxaldehyd, 3,7-Dimethyl-2-methylene-6-octenal,Phenoxyacetaldehyd; 5,9-Dimethyl-4,8-decadienal, Peony aldehyd (6,10-dimethyl-3-oxa-5,9-undecadien-1-al),Hexahydro-4,7-methanoindan-1-carboxaldehyd, Octanal, 2-Methyl octanal, alpha-Methyl-4-(I-methylethyl)benzeneacetaldehyd,6,6-Dimethyl-2-norpinene-2-propionaldehyd, para Methyl phenoxy acetaldehyd, 2-methyl-3-phenyl-2-propen-1-al,3,5,5-Trimethylhexanal, Hexahydro-8,8-dimethyl-2-naphthaldehyd, 3-Propyl-bicyclo[2.2.1]-hept-5-ene-2-carbaldehyd,9-Decenal, 3-Methyl-5-phenyl-1-pentanal, Methyl-nonyl acetaldehyd, 1-p-Menthene-q-carboxaldehyd,Citral oder Gemische davon, Lilial citral, 1-Decanal, n-Undecanal, n-Dodecanal, Florhydral, 2,4-Dimethyl-3-cyclohexen-1-carboxaldehyd 4-Methoxybenzaldehyde, 3-Methoxy-4-hydroxybenzaldehyde,3-Ethoxy-4-hydroxybenzaldehyde, 3,4-Methylendioxybenzaldehyd und 3,4-Dimethoxybenzaldehydund Gemische davon. Wie vorstehend beispielhaft ausgeführt, können die Duftaldehyde und Duftketone eine aliphatische, cycloaliphatische, aromatische, ethylenisch ungesättigte Struktur oder eine Kombination dieser Strukturen aufweisen. Es können fernerweitere Heteroatome oder polycyclische Strukturen vorliegen. Die Strukturen können geeignete Substituenten wie Hydroxyl- oder Aminogruppen aufweisen. Für weitere geeignete Duftstoffe, ausgewählt aus Aldehyden und Ketonen, wird auf Steffen Arctander "Published 1960 and 1969 respectively, Reprinted2000 ISBN: Aroma Chemicals Vol. 1: 0-931710-37-5, Aroma Chemicals Vol. 2: 0-931710-38-3", verwiesen.

Geeignete Riechstoffverbindungen vom Typ der Ester sind beispielsweise Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Riechstoffverbindungen vom Typ der Kohlenwasserstoffen sind z.B. Terpene wie Limonen und Pinen. Geeignete Duftstoffe vom Typ Ether sind beispielsweise Benzylethylether und Ambroxan. Geeignete Duftstoffalkohole sind beispielsweise 10-Undecen-1-ol, 2,6-Dimethylheptan-2-ol, 2-Methylbutanol, 2-Methylpentanol,2-Phenoxyethanol, 2-Phenylpropanol, 2-tert-Butycyclohexanol, 3,5,5-Trimethylcyclohexanol,3-Hexanol, 3-Methyl-5-phenylpentanol, 3-Octanol, 1-Octen-3-ol, 3-Phenylpropanol,4-Heptenol, 4-Isopropylcyclohexanol, 4-tert-Butycyclohexanol, 6,8-Dimethyl-2-nonanol,6-Nonen-1-ol, 9-Decen-1-ol, alpha-Methylbenzylalkohol, alpha-Terpineol, Amylsalicylat,Benzylalkohol, Benzylsalicylat, beta-Terpineol, Butylsalicylat,Citronellol, Cyclohexylsalicylat, Decanol, Dihydromyrcenol, Dimethylbenzylcarbinol,Dimethylheptanol, Dimethyloctanol, Ethylsalicylat, Ethylvanilin, Anethol, Eugenol,Geraniol, Heptanol, Hexylsalicylat, Isoborneol, Isoeugenol, Isopulegol, Linalool,Menthol, Myrtenol, n-Hexanol, Nerol, Nonanol, Octanol, para-Menthan-7-ol, Phenylethylalkohol,Phenol, Phenylsalicylat, Tetrahydrogeraniol, Tetrahydrolinalool, Thymol, trans-2-cis-6-Nonadienol,trans-2-Nonen-1-ol, trans-2-Octenol, Undecanol, Vanillin, Zimtalkohol, wobei, wenn mehrere Duftstoffalkohole vorhanden sind, diese unabhängig voneinander ausgewählt sein können.

Duftstoffe bzw. Parfümöle können auch natürliche Riechstoffgemische sein, wie sie aus pflanzlichen Quellen zugänglich sind, z.B. Pine-, Citrus-, Jasmin-, Patchouly-, Rosen- oder Ylang-Ylang-öl. Ebenfallsgeeignet sind Muskateller-Salbeiöl, Kamillenöl, Nelkenöl, Melissenöl,Minzöl, Zimtblaetteröl, Lindenbluetenöl, Wacholderbeeröl, Vetiveröl, Olibanumöl, Galbanumöl und Labdanumöl sowie Orangenblütenöl, Neroliöl, Orangenschalenöl und Sandelholzöl. Ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limette-öl, Mandarinenoel, Melissenöl, Moschuskerneröl, Myrrhenöl, Nelkenöl, Neroliöl, Niaouliöl, Olibanumöl, Origanumöl, Palmarosaöl, Patchuli-öl,Perubalsamöl, Petitgrainöl, Pfefferöl, Pfefferminzöl, Pimentöl, Pine-öl, Rosenöl, Rosmarin-öl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-öl, Ysop-öl, Zimtöl, Zimtblätteröl, Zitronellöl, Zitronenöl sowie Zypressenöl.

Ebenfalls als Duftstoff geeignet sind sogenannte Duftstoffvorläufer (Pro-Drug). Bei dieser Klasse von Verbindungen handelt es sich um Verbindungen, welche durch das Aufbrechen einer chemischen Bindung, beispielsweise durch Hydrolyse, ein erwünschtes Geruchs- und/oder Duftstoffmolekül freisetzt. Typischerweise wird zur Bildung eines Duftstoffvorläufers ein gewünschtes Duftstoffrohmaterial chemisch mit einem Träger, vorzugsweise einem geringfügig flüchtigen oder mäßig flüchtigen Träger, verbunden. Die Kombination führt zu einem weniger flüchtigen und stärker hydrophoben Duftstoffvorläufer mit verbesserter Anlagerung auf Stoffen. Der Duftstoff wird danach durch Aufbrechen der Bindung zwischen dem Duftstoffrohmaterial und dem Träger freigesetzt, beispielsweise durch eine Veränderung des pH-Werts (z. B. durch Transpiration beim Tragen), Luftfeuchtigkeit, Wärme und/oder Sonnenlicht während der Lagerung oder des Trocknens auf der Wäscheleine.

Das Duftstoffrohmaterial für Verwendung in Duftstoffvorläufern sind typischerweise gesättigte oder ungesättigte, flüchtige Verbindungen, die einen Alkohol, einen Aldehyd und/oder eine Ketongruppe enthalten. Zu den hierin nützlichen Duftstoffrohmaterialien gehören jegliche wohlriechenden Substanzen oder Mischungen von Substanzen, die bereits oben beschrieben wurden.

Besondere vorteilhafte, einsetzbare Duftstoffvorläufer gehorchen der Formel (I)

$$R\text{-}C(OR^1)(OR^2)\text{-}OR^3 \qquad (I)$$

worin R Wasserstoff, lineares $C_1$-$C_8$-Alkyl, verzweigtes $C_3$-$C_{20}$-Alkyl, cyclisches $C_3$-$C_{20}$-Alkyl, verzweigtes cyclisches $C_6$-$C_{20}$-Alkyl, lineares $C_6$-$C_{20}$-Alkenyl, verzweigtes $C_6$-$C_{20}$-Alkenyl, cyclisches $C_6$-$C_{20}$-Alkenyl, verzweigtes cyclisches $C_6$-$C_{20}$-Alkenyl, substituiertes oder unsubstituiertes $C_6$-$C_{20}$-Aryl und Mischungen hiervon bedeutet; $R^1$, $R^2$ und $R^3$ unabhängig lineares, verzweigtes oder substituiertes $C_1$-$C_{20}$-Alkyl; lineares, verzweigtes oder substituiertes $C_2$-$C_{20}$-Alkenyl; substituiertes oder unsubstituiertes, cyclisches $C_3$-$C_{20}$-Alkyl; substituiertes oder substituiertes $C_6$-$C_{20}$-Aryl, substituiertes oder unsubstituiertes $C_2$-$C_{40}$-Alkylenoxy; substituiertes oder unsubstituiertes $C_3$-$C_{40}$-Alkylenoxyalkyl; substituiertes oder unsubstituiertes $C_6$-$C_{40}$-Alkylenaryl; substituiertes oder unsubstituiertes $C_6$-$C_{32}$-Aryloxy; substituiertes oder unsubstituiertes $C_6$-$C_{40}$-Alkylenoxyaryl; $C_6$-$C_{40}$-Oxyalkylenaryl und Mischungen hiervon bedeuten. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer, sind Acetale oder Ketale, vorzugsweise gehorchend der Formel (II)

$$R\text{-}C(R^1)(OR^3)\text{-}OR2 \qquad (II)$$

worin R lineares $C_1$-$C_{20}$-Alkyl, verzweigtes $C_3$-$C_{20}$-Alcyl, cyclisches $C_6$-$C_{20}$-Alkyl, verzweigtes cyclisches $C_6$-$C_{20}$-Alkyl, lineares $C_2$-$C_{20}$-Alkenyl, verzweigtes $C_3$-$C_{20}$-Alkenyl, cyclisches C6-C20-Alkenyl, verzweigtes cyclisches $C_6$-$C_{20}$-Alke-

nyl, substituiertes oder unsubstituiertes $C_6$-$C_{20}$-Aryl und Mischungen hiervon ist; $R^1$ Wasserstoff oder R ist; $R^2$ und $R^3$ jeweils unabhängig voneinander gewählt sind aus der Gruppe, bestehend aus lineares $C_1$-$C_{20}$-Alkyl, verzweigtes $C_3$-$C_{20}$-Alkyl, cyclisches $C_3$-$C_{20}$-Alkyl, verzweigtes cyclisches $C_6$-$C_{20}$-Alkyl, lineares $C_6$-$C_{20}$-Alkenyl, verzweigtes $C_6$-$C_{20}$-Alkenyl, cyclisches $C_6$-$C_{20}$-Alkenyl, verzweigtes cyclisches $C_6$-$C_{20}$-Alkenyl, $C_6$-$C_{20}$-Aryl, substituiertes $C_7$-$C_{20}$-Aryl und Mischungen hiervon. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer gehorchen der Formel (III)

$$R^4O\text{-}C(OR^1)(OR^3)\text{-}OR^2 \qquad (III)$$

worin $R^1$, $R^2$, $R^3$ und $R^4$ unabhängig voneinander lineares, verzweigtes oder substituiertes $C_1$-$C_{20}$-Alkyl; lineares, verzweigtes oder subsituiertes $C_2$-$C_{20}$-Alkenyl; substituiertes oder unsubstituiertes, cyclisches $C_5$-$C_{20}$-Alkyl; substituiertes oder unsubstituiertes $C_6$-$C_{20}$-Aryl, substituiertes oder unsubstituiertes $C_2$-$C_{40}$-Alkylenoxy; substituiertes oder unsubstituiertes $C_3$-$C_{40}$-Alkylenoxyalkyl; substituiertes oder unsubstituiertes $C_6$-$C_{40}$-Alkylenaryl; substituiertes oder unsubstituiertes $C_6$-$C_{32}$-Aryloxy; substituiertes oder unsubstituiertes $C_6$-$C_{40}$-Alkylenoxyaryl; $C_6$-$C_{40}$-Oxyalkylenaryl; und Mischungen hiervon sind. Der Einsatz solcher Substanzen, insbesondere in (vorzugsweise wasserunlöslichen) Mikrokapseln, entspricht einer bevorzugten Ausführungsform der Erfindung.

Besonders bevorzugt ist es, wenn die eingesetzten Riechstoffe Kieselsäureester-Mischungen umfassen. Kieselsäureester werden beispielsweise durch die Formel (IV)

$$R\text{-}(\text{-O-Si }(OR)_2\text{-})_n\text{-OR} \qquad (IV)$$

beschrieben, wobei R unabhängig voneinander ausgewählt ist aus der Gruppe, die H, die geradkettigen oder verzweigten, gesättigten oder ungesättigten, substituierten oder unsubstituierten $C_1$-$C_6$-Kohlenwasserstoffreste und die Duftstoffalkoholreste und/oder Biozidalkoholreste enthält, und m Werte aus dem Bereich 1 bis 20 und n Werte aus dem Bereich 2 bis 100 annimmt. Vorzugsweise enthalten die Kieselsäureester der Formeln zumindest einen Duftstoffalkoholrest und/oder Biozidalkoholrest.

Besonders geeignete Duftstoffvorläufer sind Reaktionsprodukte von Verbindungen, die mindestens eine primäre und/oder sekundäre Amingruppe umfassen, beispielsweise einem aminofunktionellen Polymer, insbesondere einem aminofunktionellen Silikon, und einem Duftstoffbestandteil, der aus Keton, Aldehyd und Mischungen davon ausgewählt ist.

Besonders bevorzugt ist eine Lösungsmittel/Wirkstoffkombination die als Lösungsmittelmischung Propylencarbonat/Isopropylalkohol in Kombination mit mindestens einem der Wirkstoffe Citral, Phenylacetat, Limonen und Hexylacetat enthält.

## Antimikrobielle Wirkstoffe

Antimikrobielle Wirkstoffe, die ebenfalls als Aktivstoffe eingesetzt und über die erfindungsgemäßen Systeme freigesetzt werden können, umfassen beispielweise alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlor-phenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Es können jedoch auch Enzyminhibitoren wie beispielsweise Esteraseinhibitoren eingesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

Schließlich kommen auch Geruchsabsorber in Frage, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Insektenrepellentien**

Als weitere Gruppe von Aktivstoffen kommen auch Stoffe in Betracht, die Insekten abschrecken, wie beispielweise N,N-Diethyl-m-toluamid, 1,2-Pentandiol oder Ethyl Butylacetylaminopropionate.

**VERDUNSTUNGSRATE**

In einer weiteren bevorzugten Ausführungsform der Erfindung ist die Verdunstungsrate V der Aktivstoffe bzw. speziell der Duftstoffe mindestens doppelt so groß wie die Verdunstungsrate der Lösungsmittel. Unter der Verdunstungsrate V wird erfindungsgemäß der auf die Oberfläche bezogene Massenverlust innerhalb eines definierten Zeitraums unter definierten und identischen Bedingungen verstanden:

$$V = \frac{m\,(t0) - m(t)}{A_P * t}$$

Dabei bedeuten m (t0) die Masse der Probe zu Versuchsbeginn, m(t) die Masse der Probe nach der Verdunstungszeit t, $A_P$ die Oberfläche des Lösungsmittels bzw. der Duftstoffe und t der Zeitraum, in dem die Verdunstung stattgefunden hat. Zur Gewährleistung einer Vergleichbarkeit sind $A_P$, t und m (t0) konstant. Als Referenz wird die folgende Methode angewendet: 40 mg der des Lösungsmittels oder der Duftstoffe werden auf eine flache Probenpfanne des DVS 1000 der Firma SMS Surface Measurement Systems UK gegeben. Die Massenverluste der Probe werden bei 40°C ermittelt.

Der Membran-Permeationskoeffizient der Duftstoffe ist bevorzugt mindestens doppelt so groß wie der Membran-Permeationskoeffizient der Lösungsmittel. Unter dem Membran-Permeationskoeffizienten M wird erfindungsgemäß der flächenbezogene Massenstrom der Lösungsmittel bzw. der Duftstoffe durch die semipermeable Membran verstanden.

$$M = \frac{m\,(t0) - m(t)}{A_M * t}$$

Darin bedeuten m (t0) die Masse der Probe zu Versuchsbeginn, m(t) die Masse der Probe nach der Verdunstungszeit t, AM die Oberfläche der Membran und t der Zeitraum, in dem die Verdunstung stattgefunden hat. Zur Gewährleistung einer Vergleichbarkeit sind AM, t und m (t0) konstant.

Als Referenz zur Bestimmung des Membran-Permeationskoeffizienten M wird die folgende Methode angewendet: 20g Lösungsmittel bzw. Duftstoffe werden in eine Petrischale aus Glas gegeben. Die semipermeable Membran wird so auf den Rand der Petrischale aufgeklebt, dass die Öffnung der Petrischale vollständig mit der Membran bedeckt ist und der Klebstoff die Anordnung so abdichtet, dass das Lösungsmittel bzw. die Duftstoffe nicht seitlich entweichen können. Nach 2 Wochen Lagerung bei 25 °C wird der Massenverlust und M gemäß der Gleichung bestimmt.

**FARBSTOFFE**

In manchen Fällen ist es wünschenswert, wenn die erfindungsgemäßen Systeme eine Unterstützung bei der optischen Wahrnehmung der Endpunktsanzeige durch Phasentrennung erfahren. Dies kann durch Zugabe von Farbstoffen erfolgen.

Für diese Erfindung geeignete Farbstoffe müssen, außer der Löslichkeit in der jeweiligen Komponente der Zusammensetzung, keine weiteren besonderen Eigenschaften wie z.B die pH-Abhängigkeit aufweisen. Bei der vorliegenden Erfindung werden die Farbstoffe nur zur Unterstützung der optischen Wahrnehmung der Phasentrennung eingesetzt und nicht als Indikator-Farbstoffe.

Ein wesentlicher Vorteil der Erfindung ist die Eignung von besonders umweltfreundlichen Lebensmittelfarbstoffen.

In einer ersten Ausführungsform enthalten die Systeme einen einzelnen Farbstoff, der nur in einem der beiden Lösungsmittel A oder B löslich ist. Solange die lösungsvermittelnde Wirkung besteht, ist die Lösung beispielsweise blau oder grün gefärbt. Nach Erschöpfung des Aktivstoffes bilden sich zwei Phasen, von denen eine Farblos und die andere hingegen gefärbt ist.

In einer alternativen Ausführungsform enthalten die Systeme zwei unterschiedliche Farbstoffe, die jeweils nur in einem der beiden Lösungsmittel A oder B löslich sind. Solange die lösungsvermittelnde Wirkung besteht, weist die Lösung eine Mischfarbe auf, bei Endpunktsanzeige zeigen die beiden Phasen dann unterschiedliche Färbungen. Geeignete Systeme sind beispielsweise in der folgenden Tabelle C wiedergegeben:

**Tabelle C**

| Lösungsmittel/Farbstoff Systeme | | | | |
|---|---|---|---|---|
| **System** | **Lösungsmittel** | **Farbstoffe** | **Einzelfabe** | **Farbmischung** |
| I | Decamethylcyclopentasiloxan | Beta Carotin | gelb | grün |
| | Dipropylenglykol | Türkis | blau | |
| II | Isopropylmyristat | Beta Carotin | gelb | orange |
| | Propylencarbonat | Rhodamin EB4 | rot | |
| III | Isopropylmyristat | Türkis | blau | violett |
| | Propylencarbonat | Rhodamin EB4 | rot | |
| IV | Isopropylmyristat | Beta Carotin | gelb | grün |
| | Propylencarbonat | Patentblau | blau | |

Die Farbstoffe sind vorzugsweise so auszuwählen, dass diese in der ganzen Gebrauchsdauer (in jeglichem Verdampfungsstadium) gelöst vorliegen und keine sichtbare Partikel durch Ausfallen bilden.

Optional kann die Zusammensetzung einen oder mehrere Farbstoffstabilisatoren enthalten, wie z. B. Neo Heliopan BB, BHT, lonol oderTinogard Q, wobei die Einsatzmengen etwa 10 bis etwa 50 Gew.-% bezogen auf den Farbstoff betragen kann.

Folgende Farbstoffe gehören aufgrund von geeigneten Lösungseigenschaften zu bevorzugten Farbstoffen für die vorliegende Erfindung:

Pinacryptol yellow
ChromaZone Powder Red 47°C
COPPER CHLOROPHYLL OS 18%
Glowbug Red TH50
Glowbug Turquoise TH40
Macrolex Rot EG
Bekro Blue 505-0004
Bekro Red 348-0001
Bekro 74/X2032-6
Bekro 10/X2031-6
Lycopene
Savinyl feuerrot
Savinyl gelb
Savinyl blau
Solvaperm Blau 2B
Solvaperm Rot PFS
Solvaperm Rot BB
Solvaperm Rot G
Solvaperm Rot 2G
Hostasol Rot 5B
COLOR FD&C BLUE #104 (C.I.61568)
KSMFST.BLAUVIOLETTC.I.60725.
KOSMFST. TUERKIS C.I.61565
KOSMFST. TUERKIS C.I.61565
HELLGELB C.I. 12700
KOSMFST. BRAUN (C.I.12010)
KOSMFST. BRAUN (C.I.12010)
BLAUC.I.61554
KOSMFST. ALIZARIN BRILLANT VIOLETT R (C.I.60730)
COLOR BLACK C.I.77266
Rhodamin EB4 C.I.45100
KOSMFST.PHLOXIN C.I.45410
D&CRED NO. 33 C.I.17200
D & C ORANGE NO. 4/C.I.15510
ORANGE II (BLUETENORANGE, C.I.15510)
Grün (C.I.74260)
D & C GREEN NO.5 C.I.61570
KOSMFST.URANIN C.I.45350
GELB (C.I.11680)
METANILGELB C.I.13065 (ACID YELLOW 36)
KOSMFST.BLAU C.I.74160
DRAGOCOLOR PATENTBLAU VF ACID BLUE 1 CI 42045
Kosmfst. Saeureblau7, C.I.42080
KOSMFST.BLAU (C.I.61585)
LBMFST.EISENOXIDGELB E172/C.I.77492
LBMFST.AZORUBINLACK E122/C.I.14720 plv.
LBMFST.GELBORANGE-LACK E110/C.I.15985
LBMFST.CHINOLINGELBLACK E104/C.I.47005
LBMFST.TARTRAZIN-LAKE E102/C.I.19140
LBMFST.INDIGOTIN-LACK E132/C.I. 73015
LBMFST.CARMINROT E120 /C.I.75470 PLV.
LBMFST.ENOCYANIN E163
LBMFST.CHLOROPHYLL(GEKUPFERT)E141
LBMFST.CHLOROPHYLL(GEKUPFERT)E141
LBMFST.BETACAROTIN .E160A
LBMFST.ANNATTOGELB E160b/C.I.75120

LBMFST.TROCKENCOULEUR E150c
FD & C GREEN NO.3(C.I.42053)
LBMFST.BRILLANTSAEUREGRUEN E142
D & C YELLOW NO.10(C.I.47005)
FD&C YELLOW NO. 6 (C.I. 15985) E 110
FD&C YELLOW NO.5 C.I.19140
KSMFST.ECHTGELB C.I.13015
LBMFST.GRÜN 114 (E102+E133)
LBMFST.KAFFEEBRAUN E110+E102+E151+E124
LBMFST.GOLDBRAUN(E102,E110,E151)
LBMFST. BRAUN (E102,E122,E151
LBMFST.BRILLANTBLAU FCF E133/C.I.42090
KSMFST ROT C.I. 12490
RED C.I.28160
KOSMFST.BRILLANTROSA(C.I.18050)
KSMFST.GELB E(C.I.18965)
SANDOLAN WALKBLAU N-BL C.I.61585)
KOSMFST.BLAUGRÜN C.I.61570
GRUEN POV FLUORESZIEREND (C.I.42090,59040
KSMFST.FICHTENNADELGRÜN (C.I.19140,42090)
KSMFST. MAIGRÜN C.I.47005,61570
GRUENGELB FLUORESZIERENDC.I.59040
KSMFST.DIAMANTGELB fluoresz.C.I.45350
COSMETIC COLOR BLUEPASTE C.I.42051
KSMFST.EDELBLAU(C.I.42090)
EDELBLAU(C.I.42045)
FOOD COLOUR BLACK
LBMFST.TIEFSCHWARZ(E151+E110+E102)
COLOR BLACK C.I.77266
FD & C RED NO. 4, C.I.14700 Z
SYMCOLOR SPIRULINA
Symcolor LBMFST Paprikaextr. E160C
Symcolor Paprikaextr. 100.000FE
D&C Green No.6 C.I.61565
D&C RED NO.17 / C. I. 26100
Symcolor LBMFST. Annattoextr. C.I. 75120 E160B
Symcolor LBMFST. Lutein 10% E161B C.I. 75136
D&C RED NO.30 C.I.73360
LBMFST.CHLOROPHYLLINgekupfert E141
LBMFST. CITRONENGELB E102
FARBE ALLURA ROT AC 85% E129
LBMFST.BRILLANTSCHWARZ BN (E151C.I.28440)
FD&C BLAU NO.1 C.I.42090MIT E 133
LBMFST.PONCEAU 4R E124/C.I..16255
FOOD COLOR INDIGOTIN E132 C.I.73015
LBMFST.TARTRAZIN E102/C.I.19140
FD&C RED NO.40 C.I.16035 ZERTIFIZIERT
LBMFST.PATENTBLAU V(E131/C.I.42051)
LBMFST.AZORUBIN(E122/C.I.14720)
LBMFST.AMARANTH(E123/C.I.16185)
LBMFST.GELBORANGE E110/ C.I.15985
LBMFST.CHINOLINGELB /E104/C.I.47005
Beta Carotin
Azorubin
Rhodamin EB4

**FREISETZUNGSMECHANISMEN**

Bei den Systemen der vorliegenden Erfindung handelt es sich vorzugsweise - jedoch nicht ausschließlich - um Raumerfrischungssysteme, beispielsweise so genannte Membran Air Freshner, elektrische Air Freshner, Docht-Air Freshner, Rattanstab-Air Freshner und dergleichen. Diese werden üblicherweise in Behältern oder Containern angeboten, die aus Glas, Keramik oder auch Kunststoff gefertigt sind und entweder ganz oder teilweise durchsichtig sind. Dabei kann ein solcher Behälter auch so beschaffen sein, dass er grundsätzlich undurchsichtig ist, jedoch ein Fenster enthält, über welches die Endpunktsanzeige verfolgt werden kann.

Den Systemen gemeinsam ist, dass sie einen Mechanismus aufweisen müssen, der die Abgabe des Aktivstoffs, also vorzugsweise des Duftstoffs, an die Umgebung erlaubt, jedoch verhindert, dass andere volatile Komponenten, wie beispielsweise die Lösungsmittel oder unter Umständen auch die Farbstoffe in nennenswertem Umfang entweichen.

In einer ersten Ausführungsform der Erfindung handelt es sich bei dem Mechanismus zur Freisetzung des Wirkstoffes, jedoch zur Rückhaltung der weiteren volatilen Bestandteile, um eine semipermeable Kunststoffmembran.

In einer weiteren Ausführungsform der Erfindung handelt es sich bei dem Mechanismus zur Freisetzung des Wirkstoffes, jedoch zur Rückhaltung der weiteren volatilen Bestandteile, um Holz. Genauer gesagt handelt es sich um einen Behälter aus Glas, Keramik oder Kunststoff, der eine enge Öffnung aufweist, in die Holzstäbchen, beispielsweise aus Rattan eintauchen. Aufgrund der Kapillarkräfte dringen die Duftstoffe in die Holzstäbchen ein und werden an die Raumluft abgegeben, während die weniger flüchtigen Lösungsmittel - gegebenenfalls durch Einstellen der Rheologie unter Zugabe von Verdickungsmitteln - zurückbleiben. Hier ist besonders darauf zu achten, dass im Fall einer zusätzlichen Farbstoffanzeige die Farbstoffe so ausgewählt werden, dass sie nicht oder nur in geringem Umfang in das Holz diffundieren.

Die erfindungsgemäßen Systeme lasen sich lagern, bevor Lösungsmittel oder Duftstoff an die Luft abgegeben werden. Der Lagerzeitraum kann einen mindestens einen Tag, bevorzugt mindestens eine Woche, weiter bevorzugt mindestens einen Monat und insbesondere bevorzugt mindestens ein Jahr betragen. Die Temperatur während der Lagerung kann zwischen 0 und 50°C erfolgen, bevorzugt zwischen 15 und 30°C. Es ist dabei nicht notwendig eine konstante Temperatur einzuhalten. In der bevorzugten Ausführung der Erfindung wird der Geruch der Mischung oder/und die Farbe während der Lagerung nicht verändert. Zur Beurteilung einer Farb- oder/und einer Geruchsveränderung kann insbesondere der Duo-Trio Test gemäß DIN EN ISO 10399:2010-06 herangezogen werden.

**GEWERBLICHE ANWENDBARKEIT**

Ein weiterer Gegenstand der Erfindung betrifft ein Verfahren zur optischen Endpunktsanzeige für ein Wirkfreisetzungssystem, bei dem man in einem geeigneten Behälter zwei nicht oder nur geringfügig miteinander mischbare Lösungsmittel A und B vorlegt und mit einer solchen Menge eines geeigneten lösungsvermittelnden Wirkstoffes versetzt, dass eine einphasige Zubereitung entsteht, wobei die Erschöpfung des obengenannten Wirkstoffes durch Abgabe an die Umgebung dadurch angezeigt wird, dass die lösungsvermittelnde Wirkung entfällt und sich die Phasen entmischen.

In zwei unabhängig voneinander bevorzugten Ausführungsformen des Verfahrens kann man den Zubereitungen

(i) einen Farbstoff zusetzen, der nur in einem der beiden Lösungsmittel A oder B löslich ist, so dass die Endpunktanzeige durch Phasentrennung durch Bildung einer farblosen und einer gefärbten Phase unterstützt wird oder

(ii) zwei unterschiedliche Farbstoffe zusetzen, die jeweils nur in einem der beiden Lösungsmittel A oder B löslich sind, so dass die Endpunktsanzeige durch Phasentrennung durch Bildung von zwei unterschiedlich gefärbten Phasen unterstützt wird.

Ein letzter Gegenstand der Erfindung betrifft die Verwendung des eingangs geschilderten Systems als Indikator zur Verfolgung des zeitlichen Verlaufs von physikalischen und/oder chemischen Prozessen, wie beispielsweise die Verdampfung eines volatilen Stoffes, speziell eines Duftstoffes.

**BEISPIELE**

**Beispiele 1 bis 4**

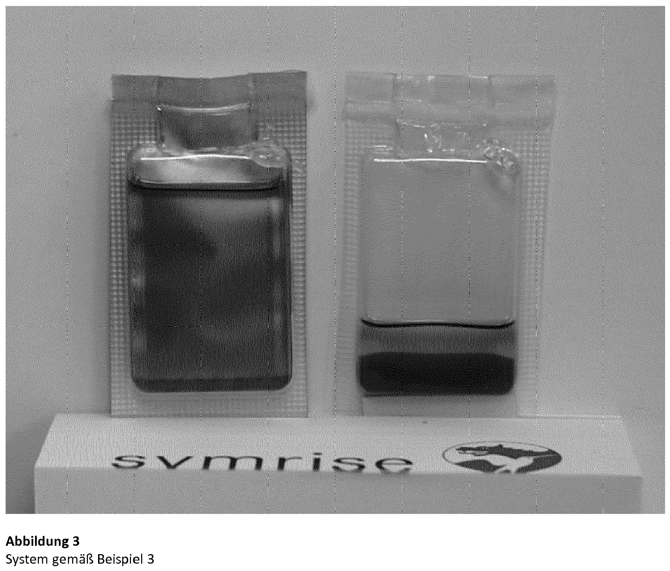

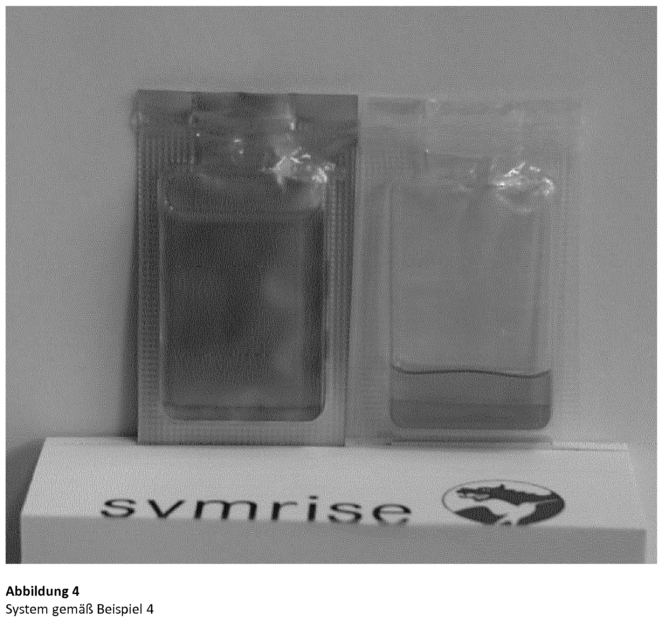

Es wurden 4 verschiedene Systeme hergestellt. Von jeder Zusammensetzung wurden je zwei Muster hergestellt. Ein Muster diente als "Startpunkt", dieses wurde im Kühlschrank gelagert und nicht geöffnet. Das zweite Muster diente als "Endpunkt, dieses wurde geöffnet und verdampft. Die Zusammensetzung der Pulver ist in **Tabelle 1** wiedergegeben. Eine optische Wiedergabe der Muster im originalzustand (einphasig) und nach Erreichen des Endpunktes findet sich in den **Abbildungen 1 bis 4.**

**Tabelle 1**

| Zusammensetzung Formulierungen | | | | |
|---|---|---|---|---|
| **Komponenten** | **1** | **2** | **3** | **4** |
| *Lösungsmittel* | | | | |
| Propylencarbonat | 3,0 | 1,5 | - | - |
| Isopropylmyristat | 1,5 | 1,5 | - | 2,0 |
| Decamethylcyclopentasiloxan | - | - | 4,0 | - |
| Dipropylenglykol | - | - | 1,0 | 4,0 |
| *Duftstoffe* | | | | |
| Citral | 0,5 | 0,5 | - | - |
| Phenylacetat | 4,0 | 4,0 | - | - |
| Limonen | 2,0 | 2,0 | - | - |
| Hexylacetat | 1,0 | 1,0 | - | - |
| Apfel CC (enthält 20 % DPG) | - | - | 15,0 | - |
| Exotic Mango New | - | - | - | 14,0 |
| *Farbstoffe* | | | | |
| Beta Carotin | 0,05 | 0,05 | 0,05 | 0,05 |
| Azorubin | 0,05 | - | - | - |
| Patentblau | - | 0,05 | - | - |
| Türkis | - | - | 0,05 | - |
| Rhodamin EB4 | - | - | - | 0,05 |

**Patentansprüche**

1. Wirkstofffreisetzungssystem mit optischer Endpunktsanzeige, umfassend

   (a) einen Behälter zur Aufnahme der Komponenten,
   (b) zwei darin befindliche Lösungsmittel A und B, die nicht oder nur geringfügig miteinander mischbar sind,
   (c) mindestens einen Wirkstoff, der als Lösungsvermittler für die Lösungsmittel A und B dient, wobei eine einphasige Zubereitung entsteht, sowie
   (d) einen Mechanismus, der die Freisetzung des Wirkstoffes in die Umgebung ermöglicht, jedoch die anderen volatilen Bestandteile der Zusammensetzung zurück hält.

   wobei die Wirkstoffe Duftstoffe, antimikrobielle Wirkstoffe und/oder Insektenrepellentien sind, und
   wobei die mischbare Lösungsmittel A und B mit einer solchen Menge genannten Wirkstoffes versetzt wird, dass eine einphasige Zubereitung entsteht,
   und wobei durch die Erschöpfung des genannten Wirkstoffes die Phasen sich entmischen.

2. Systeme nach Anspruch 1, **dadurch gekennzeichnet, dass** sie Lösungsmittel enthalten, die ausgewählt sind aus der Gruppe, die gebildet wird von $C_6$-$C_{22}$-Fettsäure-$C_1$-$C_6$-alkylestern, $C_1$-$C_6$-Alkylcarbonaten, $C_2$-$C_4$-Alkylenglykolen sowie Siloxanen.

3. Systeme nach den Ansprüchen 1 und/oder 2, **dadurch gekennzeichnet, dass** sie als Lösungsmittelkombinationen Decamethylcyclopentasiloxan/Dipropylenglykol oder Isopropy-Imyristat/Propylencarbonat enthalten.

4. Systeme nach mindestens einem der Ansprüche 1 bis 3, **dadurch gekennzeichnet, dass** sie Duftstoffe enthalten, die ausgewählt sind aus der Gruppe, die gebildet wird von Aldehyden, Ketonen, Acetalen, Ketalen und Kieselsäureestern sowie deren Gemischen.

5. Systeme nach mindestens einem der Ansprüche 1 bis 4, **dadurch gekennzeichnet, dass** sie als Lösungsmittel/Wirkstoffkombination die Lösungsmittelmischung Propylencarbonat/Isopropylalkohol in Kombination mit mindestens einem der Wirkstoffe Citral, Phenylacetat, Limonen und Hexylacetat enthält.

6. Systeme nach mindestens einem der Ansprüche 1 bis 5, **dadurch gekennzeichnet, dass** sie weiterhin Stabilisatoren enthalten.

7. Systeme nach mindestens einem der Ansprüche 1 bis 6, **dadurch gekennzeichnet, dass** sie einen Farbstoff enthalten, der nur in einem der beiden Lösungsmittel A oder B löslich ist.

8. Systeme nach mindestens einem der Ansprüche 1 bis 7, **dadurch gekennzeichnet, dass** sie zwei unterschiedliche Farbstoffe enthalten, die jeweils nur in einem der beiden Lösungsmittel A oder B löslich sind.

9. Systeme nach mindestens einem der Ansprüche 1 bis 8, **dadurch gekennzeichnet, dass** sie einen Mechanismus zur Freisetzung des Wirkstoffes, jedoch zur Rückhaltung der weiteren volatilen Bestandteile enthalten, bei dem es sich um eine semipermeable Kunststoffmembran handelt.

10. System nach mindestens einem der Ansprüche 1 bis 9, **dadurch gekennzeichnet, dass** sie einen Mechanismus zur Freisetzung des Wirkstoffes, jedoch zur Rückhaltung der weiteren volatilen Bestandteile enthalten, bei dem es sich um Holz handelt.

11. Verfahren zur optischen Endpunktsanzeige für ein Wirkfreisetzungssystem, bei dem man in einem geeigneten Behälter zwei nicht oder nur geringfügig miteinander mischbare Lösungsmittel A und B vorlegt und mit einer solchen Menge eines geeigneten lösungsvermittelnden Wirkstoffes versetzt, dass eine einphasige Zubereitung entsteht, wobei die Erschöpfung des obengenannten Wirkstoffes durch Abgabe an die Umgebung dadurch angezeigt wird, dass die lösungsvermittelnde Wirkung entfällt und sich die Phasen entmischen.

12. Verfahren nach Anspruch 11, **dadurch gekennzeichnet, dass** man der Zubereitung einen Farbstoff zusetzt, der nur in einem der beiden Lösungsmittel A oder B löslich ist, so dass die Endpunktanzeige durch Phasentrennung durch Bildung einer farblosen und einer gefärbten Phase unterstützt wird.

13. Verfahren nach Anspruch 11, **dadurch gekennzeichnet, dass** man der Zubereitung zwei unterschiedliche Farbstoffe zusetzt, die jeweils nur in einem der beiden Lösungsmittel A oder B löslich sind, so dass die Endpunktsanzeige durch Phasentrennung durch Bildung von zwei unterschiedliche gefärbten Phasen unterstützt wird.

14. Verwendung eines Systems nach Anspruch 1 als Indikator zur Verfolgung des zeitlichen Verlaufs von physikalischen und/oder chemischen Prozessen.

**Claims**

1. System for release of active agents with optical limit point indication, comprising

   (a) a container for take-up of the components;
   (b) two solvents A and B placed therein, being not or only slightly miscible with each other;
   (c) at least one active agent, serving as a solubiliser for said solvents A and B, whereby providing a single-phase composition; and
   (d) a mechanism capable of releasing said active agents into environment, while retaining all other volatile components of said composition,

   wherein said active agents represent fragrances, antimicrobial actives and/or insect repellents; and
   wherein said miscible solvents A and B are treated with such an amount of said active agents to obtain a single-phase composition, and
   whereby said phases are separating when the amount of said active agents is exhausted.

2. Systems according to Claim 1, **characterised in that** it contains solvents selected from the group consisting of $C_6$-$C_{22}$ fatty acid $C_1$-$C_6$ alkyl esters, $C_1$-$C_6$ alkyl carbonates, $C_2$-$C_4$ alkylene glycols and siloxanes.

3. Systems according to claim 1 and/or 2, **characterised in that** they comprise decamethylcyclopentasiloxane/dipropylene glycol or isopropylmyristate/propylene carbonate as solvent combinations.

4. Systems according to any of the claims 1 to 3, **characterised in that** they comprise fragrances seklected from the group selected from aldehydes, ketones, acetales, ketales and silica acid esters and their mixtures.

5. Systems according to any of the claims 1 to 4, **characterised in that** they comprise as a solvent/active combination the solvent mixture propylene carbonate/isopropyl alcohol in combination with at least one of the actives citral, phenyl acetate, limonene and hexyl acetate.

6. Systems according to any of the claims 1 to 5, **characterised in that** they also contain stabilisers.

7. Systems according to any of the claims 1 to 6, **characterized in that** they comprise a dye which is soluble only in one of the solvents A or B.

8. Systems according to any of the claims 1 to 7, **characterized in that** they comprise two different dyes, each of them soluble only in one of the solvents A or B.

9. Systems according to any of the claims 1 to 8, **characterised in that** they comprise a mechanism for release of active agents, but retaining all other volatile components representing a semipermeable membrane.

10. Systems according to any of the claims 1 to 8, **characterised in that** they comprise a mechanism for release of active agents, but retaining all other volatile components representing wood.

11. A process for optical limit indication for a system for release of active agents, according to which two solvents A and B being not or even slightly miscible with each other are placed in a container, and are treated with such an amount of a suitable solubilising active agents to form a single-phase composition, whereby indication of the limit point of said active agent to the environment is provided by omission of the solubilising effect and separation of the phases.

12. The process of claim 11, **characterised in that** a dye is added to the composition, said dye being soluble in only one of the two solvents A or B, so that indication of the limit point is supported by forming a colourless and coloured phase.

13. The process of claim 12, **characterised in that** two different dyes are added to the composition, each of them only soluble in one of the solvents A or B, so that indication of the limit point is supported by forming two phases of different colours.

14. Use of a system according to claim 1 as an indicator for follow-up of time sequence of physical and/or chemical processes.

**Revendications**

1. Système de la libération des principes actifs avec une indication optique du point final comprenant

    (a) un récipient pour contenir des composants,
    (b) deux solvants A et B placés à l'intérieure qui sont non miscibles entre eux ou seulement légèrement miscibles,
    (c) au moins un principe actif qui opère en tant qu'agent de solubilisation pour les solvants A et B dans lequel une préparation en phase unique se forme, de même que
    (d) un mécanisme qui permet la libération de la substance dans l'environnement, mais qui néanmoins retient les autres composants volatils de la composition

    dans lequel les principes actifs sont des parfums, des antimicrobiens et/ou des répulsifs pour insectes et
    dans lequel les solvants miscibles A et B sont additionnés avec ledit principe actif dans une quantité telle qu'une préparation en phase unique se forme
et dans lequel les phases ses démixent par l'épuisement de ladite substance.

2. Systèmes selon la revendication 1, **caractérisé en ce qu'**ils contiennent des solvants choisi parmi le groupe consistant de $C_6$-$C_{22}$-Acide gras-$C_1$-$C_6$- alkyl ester, $C_1$-$C_6$-carbonates d'alkyle, $C_2$-$C_4$- alkylèneglycols de même que siloxanes.

3. Systèmes selon les revendications 1 et/ou 2, **caractérisé en ce qu'**ils contiennent le décaméthyle cyclopentasiloxane/ le dipropylèneglycol ou le myristate d'isopropyle/ le carbonate de propylène comme combinaison des solvants.

4. Systèmes selon au moins une des revendications 1 à 3, **caractérisé en ce qu'**ils contiennent des parfums choisi parmi le groupe consistant des aldéhydes, des cétones, des acétales, des cétales et des esters d'acide silicique de même que ses mélanges.

5. Systèmes selon au moins une des revendications 1 à 4, **caractérisé en ce qu'**ils contiennent le mélange du solvant carbonate de propylène/de l'alcool isopropylique en combinaison avec au moins un des principes actifs du citral, de l'acétate de phényle, des citrons verts et de l'acétate d'héxyle comme solvant/combinaison de principe.

6. Systèmes selon au moins une des revendications 1 à 5, **caractérisé en ce qu'**ils contiennent également des stabilisateurs.

7. Systèmes selon au moins une des revendications 1 à 6, **caractérisé en ce qu'**ils contiennent un colorant qui est seulement solvable dans un des deux solvants A ou B.

8. Systèmes selon au moins une des revendications 1 à 7, **caractérisé en ce qu'**ils contiennent deux colorants différents qui sont respectivement seulement solvable dans un des deux solvants A ou B.

9. Systèmes selon au moins une des revendications 1 à 8, **caractérisé en ce qu'**ils contiennent un mécanisme pour la libération du principe actif mais néanmoins pour la rétention des autres composants volatils dans lequel il s'agit d'une membrane semi-perméable en matière plastique.

10. Systèmes selon au moins une des revendications 1 à 8, **caractérisé en ce qu'**ils contiennent un mécanisme pour la libération du principe actif mais néanmoins pour la rétention des autres composants volatils dans lequel il s'agit du bois.

11. Procédé d'indication optique du point final pour un système de la libération d'effet dans lequel on soumet deux solvants A et B qui sont non miscibles entre eux ou seulement légèrement miscibles dans un récipient convenant et dans lequel lesdits solvants sont additionnés avec un principe actif solubilisant dans une quantité qu'une préparation en phase unique est née et dans lequel l'épuisement dudit principe actif par l'émission dans l'environnement est présentée en ce que l'effet solubilisant est supprimé et que les phases ses démixent.

12. Procédé selon la revendication 11, **caractérisé en ce qu'**on ajoute un colorant à la préparation qui est seulement solvable dans un des deux solvants A ou B de sorte que l'indication du point final est soutenu par la séparation des phases par formation d'une phase incolore et d'une phase coloré.

13. Procédé selon la revendication 12, **caractérisé en ce qu'**on ajoute deux colorants différents à la préparation qui sont seulement solvables dans un des deux solvants A ou B de sorte que l'indication du point final est soutenu par la séparation des phases par formation de deux phases colorés différemment.

14. Utilisation d'un système selon la revendication 1 comme indicateur pour poursuivre le déroulement chronologique des procédés physiques et/ou chimique.

EP 2 918 295 B1

**Abbildung 1**
System gemäß Beispiel 1

**Abbildung 2**
System gemäß Beispiel 2

**Abbildung 3**
System gemäß Beispiel 3

**Abbildung 4**
System gemäß Beispiel 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4128508 A
- WO 2003031966 A1
- GB 2444702 A
- WO 2004087225 A1

**In der Beschreibung aufgeführte Nicht-Patentliteratur**

- **GODFREY N.B.** Solvent selection via miscibility number. *Chemtech.,* 1972, vol. 6, 359-363